Europäisches Patentamt

European Patent Office

Office européen des brevets

(11) Veröffentlichungsnummer: **0 062 234**
A2

(12) **EUROPÄISCHE PATENTANMELDUNG**

(21) Anmeldenummer: 82102440.3

(51) Int. Cl.³: **A 61 F 13/10**

(22) Anmeldetag: 24.03.82

(30) Priorität: 27.03.81 DE 3112155

(43) Veröffentlichungstag der Anmeldung: 13.10.82
Patentblatt 82/41

(84) Benannte Vertragsstaaten: **AT BE CH FR GB IT LI NL**

(71) Anmelder: **Hildebrandt, Hans-Dietrich, Dr. med., Hohlesteinweg 16, D-3501 Ahnatal (DE)**

(72) Erfinder: **Hildebrandt, Hans-Dietrich, Dr. med., Hohlesteinweg 16, D-3501 Ahnatal (DE)**

(74) Vertreter: **Freiherr von Schorlemer, Reinfried, Brüder-Grimm-Platz 4, D-3500 Kassel (DE)**

(54) **Epicondylitis-Bandage.**

(57) Epicondylitis-Bandage, bestehend aus einem um den Unterarm zu legenden Band, das einen Funktionsabschnitt und an diesem befestigte Verschlußelemente aufweist, wobei wenigstens der Funktionsabschnitt eine an die Anatomie des Unterarms angepaßte Form besitzt.

Epicondylitis-Bandage

Die Erfindung betrifft eine Epicondylitis-Bandage der im Oberbegriff des Anspruchs 1 definierten Gattung.

Epicondylitis, häufig auch als "Tennisarm" bezeichnet, entsteht durch Überbeanspruchung einer Muskelgruppe im Ellenbogenbereich und hat heftige Schmerzen bei Druck und Belastung zur Folge. Außer bei Sportlern wie Tennis-, Tischtennis-, Badminton- oder Golfspielern tritt dieses Leiden auch bei Personen auf, die infolge beruflicher oder privater Tätigkeiten die genannte Muskelgruppe häufig überlasten.

Zur Linderung der Schmerzen sind Epicondylitis-Bandagen der eingangs bezeichneten Art bekannt, die in der Regel aus einem einfachen Gurtband bestehen, an dessen Enden Verschlußelemente, beispielsweise zur Bildung eines Klettenverschlusses vorgesehen sind, und mittels/derer der Muskelansatz der betroffenen Muskelgruppe künstlich vorverlegt und bei Benutzung der Hand entlastet werden soll.

Als nachteilig hat sich erwiesen, daß alle bekannten Epicondylitis-Bandagen zu leicht verrutschen und daher gerade bei Ausübung derjenigen sportlichen oder anderen Tätigkeiten, die ein Tragen der Bandage aus medizinischen Gründen erforderlich machen, nicht an der vorgesehenen Stelle dicht unterhalb des Ellenbogens haften bleiben. Eine Folge davon ist, daß derartige Bandagen zwar verschrieben und gekauft, aber nur selten über längere Zeiträume hinweg tatsächlich benutzt werden und daher nicht den erwünschten Erfolg mit sich bringen.

Der Erfindung liegt die Aufgabe zugrunde, eine Epicondylitis-Bandage der eingangs bezeichneten Gattung zu schaffen, die nicht rutscht und vor allem bei Ausübung der die Epicondylitis verursachenden Tätigkeiten fest und sicher am Unterarm haften bleibt.

Zur Lösung dieser Aufgabe sind die kennzeichnenden Merkmale
des Anspruchs 1 vorgesehen.

Die Erfindung bringt den Vorteil mit sich, daß durch die besondere Formgebung des Funktionsabschnitts ein Rutschen der
Bandage vollständig ausgeschaltet wird, selbst wenn sich der
Funktionsabschnitt nicht über den ganzen Umfang des Unterarms, sondern nur über einen Teil desselben erstreckt. Dieses Ergebnis überrascht insofern, als wegen der etwa konischen Verjüngung des Unterarms in Richtung der Hand bisher
allgemein angenommen wurde, daß ein Rutschen der Bandage
allenfalls durch Einsatz eines besonderen Materials, nicht
aber durch eine bestimmte Formgebung  verhindert werden
könnte.

Weitere Vorteile der Erfindung sind in den Unteransprüchen
gekennzeichnet.

Die Erfindung wird nachfolgend in Verbindung mit der beiliegenden Zeichnung an einem Ausführungsbeispiel näher erläutert. Es zeigen:

Fig. 1 eine Draufsicht auf eine erfindungsgemäße Epicondylitis-
       Bandage im abgerollten Zustand; und

Fig. 2 eine  Unteransicht der  Bandage nach Fig. 1 im Gebrauchszustand.

Die erfindungsgemäße Epicondylitis-Bandage 1 weist einen
Funktionsabschnitt 2 auf, der im Gegensatz zu bekannten
Bandagen dieser Art nicht von zwei parallelen und geradlinigen, sondern von zwei bogenförmigen, an die Anatomie
des Unterarms angepaßten Längsrändern 3 und 4 begrenzt ist.
Unter einer an die Anatomie des Unterarms angepaßten Form
wird hierbei verstanden, daß der Funktionsabschnitt 2 wie
der dicht unterhalb des Ellenbogens befindliche Teil des
Unterarms im Gebrauchszustand etwa längs einer Kegelstumpffläche verläuft und sich daher fest und gleichförmig dem
Unterarm anschmiegt. An den Enden des Funktionsabschnitts 2
sind Verschlußelemente vorgesehen, die beispielsweise aus bandförmigen Streifen 5 und 6 bestehen, von denen der eine
Schichten 7 zur Bildung eines Klettenverschlusses aufweist,
während der andere an seinem freien Ende mit einem Ring bzw.
einer Öse 8 oder dgl. versehen ist, durch die der Streifen 6
durchgezogen und dann auf sich selbst zurückgefaltet wird.

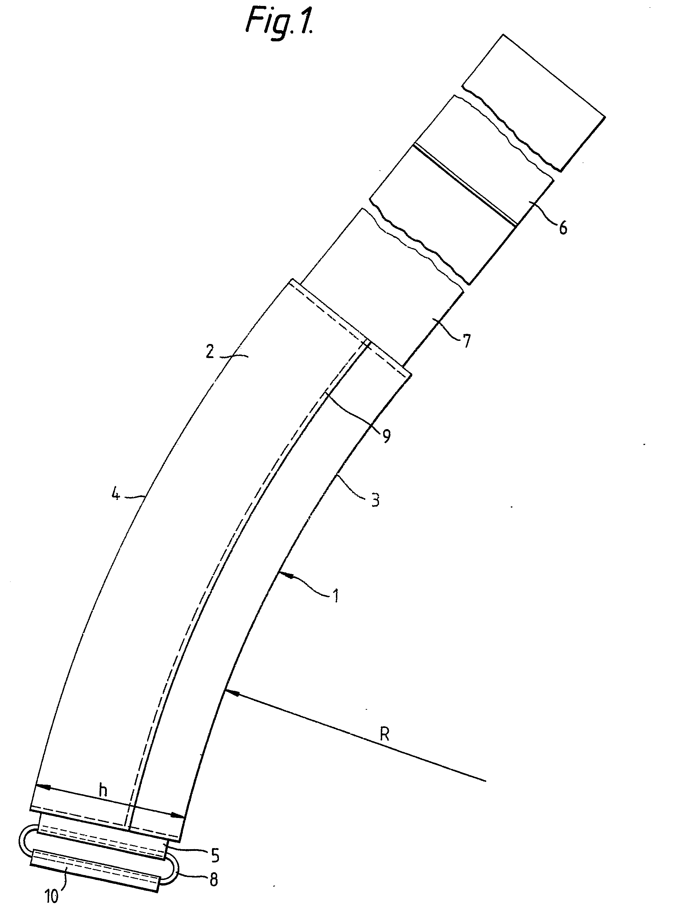

Der Funktionsabschnitt 2 braucht nicht exakt wie ein Kegelstumpfmantel geformt sein, weil bereits die durch den bogenförmigen Verlauf der Längsränder 3 und 4 erzielte Form ein
Rutschen der Bandage am Unterarm nahezu ausschließt, wenn
der Krümmungsradius der Längsränder 3 und 4 unter Berücksichtigung der Anatomie des Unterarms ausreichend groß gewählt ist. Bei einer praktischen Ausführungsform beträgt
die Länge des oberen Längsrandes etwa 16,5 cm, die Länge
des unteren Längsrandes 3 etwa 14,5 cm, die Höhe $\underline{h}$ des
Funktionsabschnitts 2 etwa 4,5 cm und der Radius $\underline{R}$ der beiden Längsränder 3 und 4 etwa 40 cm. Die Streifen 5 und 6
schließen sich an den beiden Enden des Funktionsabschnitts
2 etwa tangential an und könnten entgegen Fig. 1 ebenfalls anatomiegerecht geformt sein.

Obwohl eine Bandage 1 mit den angegebenen Dimensionen bei
beeigneter Länge der Streifen 5 und 6 praktisch für Unterarme
Größe jeder . brauchbar ist und dabei zur Behandlung sowohl der
Epicondylitis humeri radialis als auch der Epicondylitis
humeri ulnaris benutzt werden kann, wird die erfindungsgemäße Bandage 1 zweckmäßig in zwei oder mehr Größen hergestellt und geliefert, damit auch bei besonders stark oder
schwach entwickelten Unterarmen der erwünschte feste Sitz
gewährleistet ist. Außerdem kann entsprechend Fig. 1 vorgesehen sein, den beiden Längsrändern 3 und 4 unterschiedliche Krümmungsradien zu geben, so daß beispielsweise die
Höhe $\underline{h}$ des Funktionsabschnitts 2 im mittleren Bereich
etwas größer als an den beiden Enden ist.

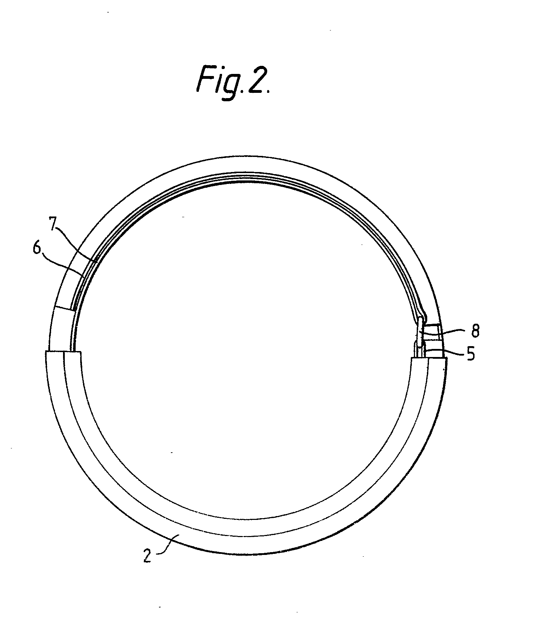

Als Materialien für den Funktionsabschnitt eignen sich
beispielsweise Leder, Kunstleder und ein von der Fa. Heinz
Ziegenbein KG, 7000 Stuttgart unter der Bezeichnung "Molliskin" vertriebenes Material, das aufgrund seiner veloursartigen, rauhen Oberfläche die Haftung der Bandage 1 am Arm zusätzlich fördert. Die beiden Streifen 5 und 6 können dabei entweder am Funktionsabschnitt 2 beispielsweise durch Nähen befestigt sein oder die Endabschnitte eines durchgehenden Bandes bilden, dessen Mittelteil mit einem Materialabschnitt derart umwickelt und beispielsweise vernäht ist, daß sich die Form nach Fig. 1 und 2 ergibt, in der das Bezugszeichen 9 eine Nähnaht andeutet. Auch andere Ausführungsformen sind denkbar. Die Streifen 5 und 6 bestehen zweckmäßig aus einem normalen Gurtband, das bei Anwendung eines durchgehenden Bandes in seinem den Funktionsabschnitt 2 tragenden Bereich zweckmäßig entsprechend bogenförmig gestaltet ist. Alternativ könnten der Funktionsabschnitt 2 und die Streifen 5,6 aus einem Stück bestehen und beispielsweise aus einem entsprechend zugeschnittenen Materialstreifen hergestellt sein.

Als Verschlußelemente können andere als die dargestellten, einen Klettenverschluß bildenden Verschlußelemente vorgesehen werden, um die Bandage am Unterarm zu fixieren. Der freie Bügel des Rings 8 kann beispielsweise eine Rolle 10 tragen, die das Festziehen des in den Ring 8 eingelegten Streifens 6 beim Anlegen der Bandage 1 erleichtert.

0062234

Patentanwalt
Diplom-Physiker
**Reinfried Frhr. v. Schorlemer**

D-3500 Kassel
Brüder-Grimm-Platz 4
Telefon (0561) 15335

D 5109

Dr. Hans-Dietrich Hildebrandt, 3501 Ahnatal

Ansprüche

1) Epicondylitis-Bandage, bestehend aus einem um den Unterarm zu legenden Band, das einen Funktionsabschnitt und an diesem befestigte Verschlußelemente aufweist, dadurch gekennzeichnet, daß wenigstens der Funktionsabschnitt (2) eine an die Anatomie des Unterarms angepaßte Form besitzt.

2) Bandage nach Anspruch 1, dadurch gekennzeichnet, daß der Funktionsabschnitt (2) längs eines Kegelstumpfmantels verläuft.

3) Bandage nach Anspruch 1 oder 2, dadurch gekennzeichnet, daß sie aus einem durchgehenden Band besteht, das mit einem den Funktionsabschnitt (2) bildenden Element umwickelt ist.

4) Bandage nach einem der Ansprüche 1 bis 3, dadurch gekennzeichnet, daß der Funktionsabschnitt (2) aus einem rutschfesten Material besteht.

5) Bandage nach einem der Ansprüche 1 bis 4, dadurch gekennzeichnet, daß die Länge des Funktionsabschnitts (2) etwa einem halben Armumfang entspricht.

1/2

*Fig.1.*

*Fig.2.*